# EUROPEAN PATENT APPLICATION

(11) **EP 4 624 571 A1**
(43) Date of publication of application: **01.10.2025**
(21) Application number: 24167062.9
(22) Date of filing: 27.03.2024
(51) Int. Cl.: C12N 9/14, C12N 1/20, A61K 35/76, A61K 38/43, A61P 17/10, A61P 31/02

(54) **ANTIBACTERIAL PROTEIN FOR CONTROL OF CUTIBACTERIUM ACNES**

(71) Applicant: UAB Nomads, Vilnius (LT)
(72) Inventor: Kazanaviciute, Vaiva, Vilnius (LT); Misiunas, Audrius, Vilnius (LT); Razanskiene, Ausra, Vilnius (LT)
(74) Representative: AAA Law

(57) **Abstract**

The present invention relates to a new isolated antibacterial protein that specifically targets *Cutibacterium acnes* and thus is able to prevent and/or inhibit the infection by *C. acnes.* Advantageously, very low concentrations (in the range of ng/ml) of the newly isolated antibacterial protein are able to prevent and/or inhibit the growth of *C. acnes.* Accordingly, an isolated antibacterial protein is provided, as well as a composition for targeting *Cutibacterium acnes* comprising said antibacterial protein. The antibacterial protein or the composition comprising the isolated antibacterial protein of the present invention for use in a method of treating or preventing infection with *Cutibacterium acnes* are provided, as well as a method for inhibiting or preventing the growth of *Cutibacterium acnes* comprising the step of contacting the bacteria with the antibacterial protein of the present invention.

## Description

### FIELD OF INVENTION

The invention relates to an isolated antibacterial protein for the control of *Cutibacterium acnes.* The invention also relates to a composition, in particular, a cosmetic, dermatological or pharmaceutical composition, comprising said antibacterial protein. The invention further relates to antibacterial protein or its composition for use in treatment or prevention of infection with *C*. *acnes.*

### BACKGROUND OF INVENTION

*Cutibacterium acnes* (formerly *Proprionibacterium acnes*) is a Gram-positive aerotolerant anaerobic bacterium. *Cutibacterium acnes* (*C. acnes*) is mainly found in the areas of the skin rich in sebaceous glands; it accounts for about 90% of the total skin microbiota making it the most abundant bacterium (Dessinioti et al. 2017). Even though being considered a skin commensal, *C*. *acnes* is associated with skin disorders such as acne, and potentially with other important diseases, for example, prostate cancer (Lomholt et al., 2010; Alexeyev et al., 2007).

Acne vulgaris is the most abundant skin disorder mainly affecting adolescents and young adults. Besides being a skin disorder, it can prove to be an emotional, psychological and social challenge causing depression (Kraft et al., 2011). *C*. *acnes* is believed to be the main contributor in the pathology of acne vulgaris where it leads to the formation of acne lesions by eliciting inflammatory response. Its conventional treatment with topical and systematic antibiotic administration has led to an upsurge in resistance and detection of antibiotic resistant *C*. *acnes* strains has become increasingly common, necessitating search of new non-conventional antimicrobials (Kim et al., 2023).

The exploitation of bacteriophage lysins, or endolysins, is one of the approaches for the treatment of infections caused by MDR (Multi Drug Resistant) bacteria. Endolysins are hydrolytic enzymes encoded by bacteriophages and degrade the peptidoglycan (PG) layer of the host bacterial cell at the end of the lytic replication cycle, causing disruption of the cell's integrity. Treatment of bacteria with endolysins provides major advantages over the administration of antibiotics, including immediate bactericidal activity, high target specificity, low toxicity to mammalian cells, and a low to minimum risk of developing resistance (Varotsou et al., 2023).

The first source for isolating bacteriophage lysin sequences are phages infecting *C*. *acnes.* These phages seem to represent a unique, almost clonal lineage of phages with low similarity to other phages. There is an astonishing conservation of different *C*. *acnes* phage genomes, with a nucleotide identity higher than 85%, despite their isolation from different countries (England, Sweden, and USA) and different times of isolation covering a time span of more than 30 years. It has been speculated that one reason for this conservation might be the specific niche of *C*. *acnes,* which reduces lateral gene transfer or recombination events (Bruggemann, Lood, 2013).

There has not been much success up to date in expressing efficient and sufficiently soluble lytic proteins for control of acne. The endolysins known up to date that could to some extent be used against *C*. *acnes* typically are small (about 30 kDa) hydrophilic proteins, containing N-acetylmuramoyl-L-alanine amidase domain. For example, recently, recombinant CAP 10-3 bacteriophage endolysin has been published, showing antibacterial activity against *C*. *acnes* in a dose-dependent manner. However, the solubility levels and purification yields achieved were not satisfactory in view of the suggested use of the protein (Kim et al. 2023).

In another study, (PaAmi1), derived from *Cutibacterium acnes* phage PAC1, was expressed in *E*. *coli* and demonstrated some activity against *C*. *acnes.* In order to be efficient, this lysin was further optimized by adding "artilysin" peptides and showed moderate activity not only against *C*. *acnes,* but also against a broad range of microorganisms, including gram negatives (Varotsou et al. 2023). Therefore, there remains a need for more efficient antibacterial proteins that would specifically target *Cutibacterium acnes,* and, at the same time, for proteins that can be provided in recombinant form, have good solubility and/or can be produced in high purification yields.

### SUMMARY OF INVENTION

The present invention provides a new isolated antibacterial protein that specifically targets *Cutibacterium acnes* and thus is able to prevent and/or inhibit the infection by *C*. *acnes.* Advantageously, very low concentrations (in the range of ng/ml) of the newly isolated antibacterial protein are able to prevent and/or inhibit the growth of *C*. *acnes.* Accordingly, it is possible to use the antibacterial protein according to the present invention in treatment of acne by means of eradicating or reducing skin colonization by *C*. *acnes.* In a further aspect, the antibacterial protein according to the present invention has good solubility, and, additionally, can be produced in high purification yields.

Accordingly, the present invention provides the following:
In a first aspect, an isolated antibacterial protein comprising a polypeptide comprising or consisting of a) an amino acid sequence of SEQ ID NO: 1; b) an amino acid sequence having at least 80%, 85%, 90%, 95% or 99% sequence identity to the amino acid sequence of SEQ ID NO: 1; or c) an amino acid sequence having at least 80%, 85%, 90%, 95%, 99% or 100% sequence identity to the amino acid sequence corresponding to amino acids 140-813 of SEQ ID NO: 1, is provided.

In a second aspect, a composition for targeting *Cutibacterium acnes* comprising said antibacterial protein is provided. In some embodiments, the antibacterial protein is provided at a concentration from about 100 ng/ml to about 1 mg/ml. In preferred embodiment, the antibacterial protein is provided at a concentration from about 100 ng/ml to about 100 µg/ml, preferably at a concentration of about 100 µg/ml. In another preferred embodiment, the antibacterial protein is provided at a concentration from about 100 ng/ml to about 10 µg/ml, more preferably at a concentration of about 10 µg/ml.

In a third aspect, a cosmetic, dermatological or pharmaceutical composition comprising the isolated antibacterial protein of the present invention or the composition comprising the isolated antibacterial protein of the present invention is provided. In some embodiments, said cosmetic, dermatological or pharmaceutical composition is in a form of a gel, a lotion or a cream. In some embodiments, said pharmaceutical composition comprises one or more pharmaceutically acceptable carrier.

In a fourth aspect, the antibacterial protein or the composition comprising the isolated antibacterial protein of the present invention for use in a method of treating or preventing infection with *Cutibacterium acnes* is provided.

In a fifth aspect, the antibacterial protein or the composition comprising the isolated antibacterial protein of the present invention for use in a method of treatment or prevention of acne vulgaris is provided.

In a sixth aspect, a method for inhibiting or preventing the growth of *Cutibacterium acnes* comprising the step of contacting the bacteria with the antibacterial protein of the present invention or with the composition comprising the isolated antibacterial protein of the present invention is provided.

In a seventh aspect, a nucleic acid encoding the antibacterial protein of the present invention is provided. In some embodiments, a recombinant expression vector comprising said nucleic acid encoding the antibacterial protein of the present invention is provided. In yet further embodiments, a host cell comprising said nucleic acid or said recombinant expression vector is provided.

### DESCRIPTION OF DRAWINGS

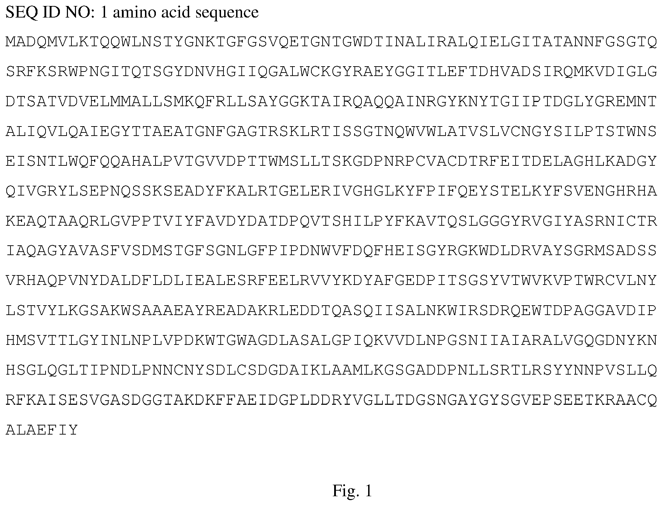

**Figure 1****.** The amino acid sequence (SEQ ID NO: 1) of ALT33441 protein.

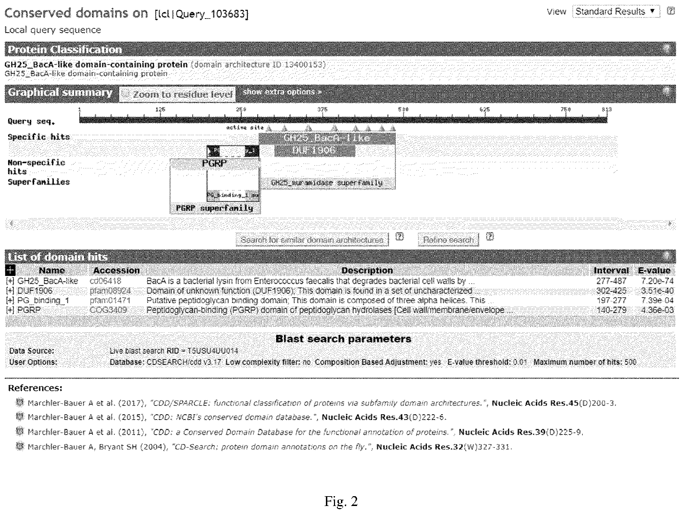

**Figure 2****.** Predicted domain structure of ALT33441.

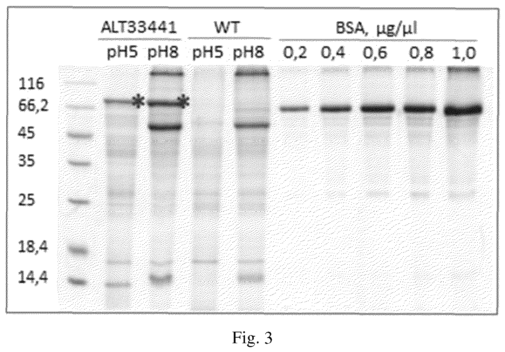

**Figure 3****.** Crude protein extracts prepared from infiltrated *N. benthamiana* leaves following the standard procedure and by using 50 mM Na phosphate, 300 mM NaCl buffer of pH 5.0 and pH 8.0. 2 µl of extracts or BSA were loaded on 12.5% SDS-PAG for electrophoresis. ALT33441 - ALT33441-infiltrated leaf extract, WT - wild-type non-infiltrated leaf extract. The asterisk shows the target protein. First lane - Pierce^{™} Unstained Protein MW Marker (Thermo Fisher Scientific).

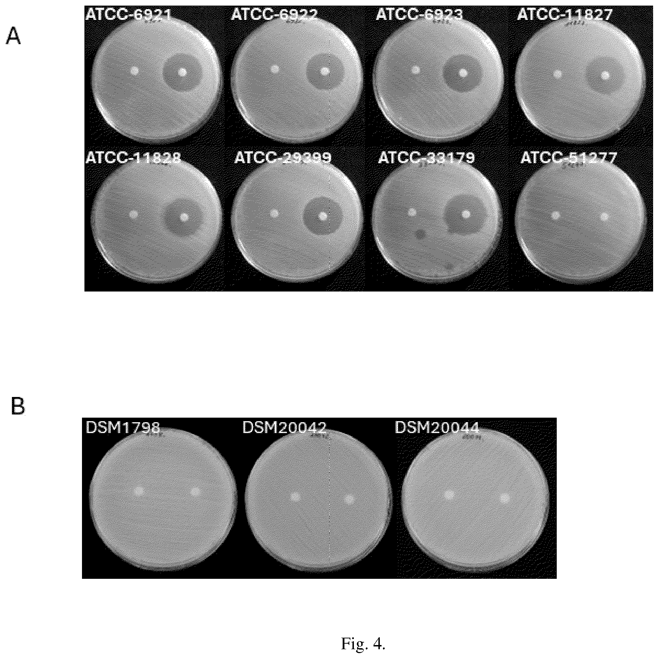

**Figure 4****.** Lytic activity detection in agar diffusion assays, using crude protein ALT33441 extracts. Crude extract spots were dropped on the plates in the following order: left disc - wild-type crude extract, right disc - ALT33441-infiltrated leaf extract. A - lytic activity detection assay on *C*. *acnes* lawns, B - lytic activity detection assay on *S*. *epidermidis* lawns.

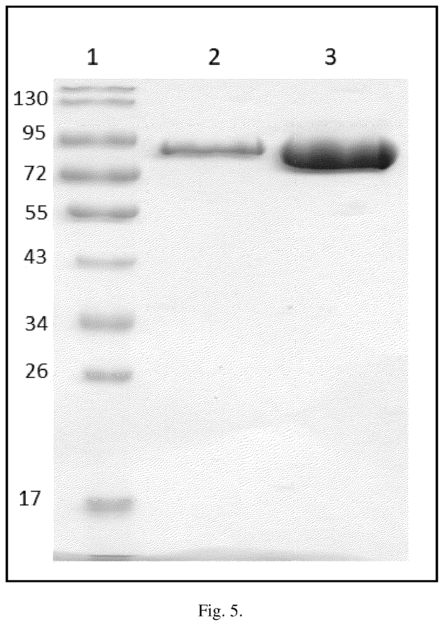

**Figure 5****.** SDS-PAGE of purified ALT33441. Lane 1 - Pierce^{™} Unstained Protein MW Marker (Thermo Fisher Scientific), lane 2- Flow-through fraction after Q sepharose FF resin column, lane 3 - concentrated flow-through fraction. 2 µl loaded on the 12.5% gel.

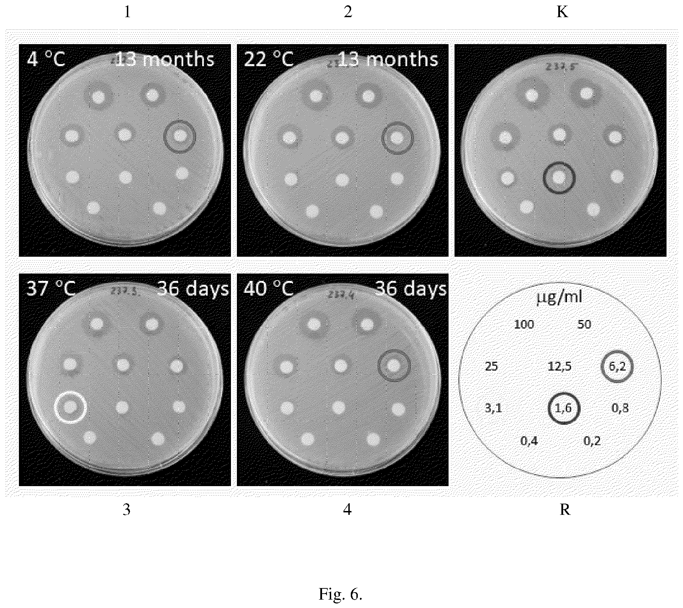

**Figure 6****.** Antibacterial activity of ALT33441 after storage at different temperatures. 1 -after storage at 4°C for 13 months, 2 - after storage at 22°C for 13 months, 3 - after storage at 37°C for 36 days, 4 - after storage at 40°C for 36 days. K - a control plate with the freshly purified ALT33441, R - a reference plate scheme with respective protein concentrations indicated. Circles around paper discs mark minimum protein concentrations where lysis of bacteria was observed.

### DETAILED DESCRIPTION OF THE INVENTION

The present inventors have identified new antibacterial protein that specifically targets *Cutibacterium acnes,* acts as a lysin and thus is able to prevent and/or inhibit the infection by *C*. *acnes.* The isolated antibacterial protein according to the present invention, referred also herein as ALT33441, comprises protein domains that differ from the protein domains of endolysins that have been to date identified to show at least minimal activity against *C*. *acnes.* Surprisingly, it was found that not only the isolated protein ALT33441 had antibacterial activity, but that it was capable of lysing *C*. *acnes* from the outside and very low concentrations (in the range of ng/ml) of the protein already had an effect in preventing and/or inhibiting the growth of *C*. *acnes.* ALT33441 specifically targets *C*. *acne* and also it was shown to have extremely low Minimal inhibitory concentration (MIC) and Minimal bactericidal concentration (MBC) against all tested sensitive strains. Advantageously, the antibacterial protein according to the present invention was shown to have good protein solubility and can be produced in high purification yields. As a further advantage, the hereby disclosed antibacterial protein has high stability and remains highly active for at least one year at room temperature (e.g. at 22°C). The antibacterial protein according to the present invention can be useful in treatment of acne by means of eradicating or reducing skin colonization by *C*. *acnes.* Alternatively, the antibacterial protein of the present invention can be useful in preventing or treating other illnesses caused by *C*. *acnes.*

Accordingly, the present invention disclosure provides an isolated antibacterial protein comprising a polypeptide comprising or consisting of a) an amino acid sequence of SEQ ID NO: 1; b) an amino acid sequence having at least 80%, 85%, 90%, 95% or 99% sequence identity to the amino acid sequence of SEQ ID NO: 1; or c) an amino acid sequence having at least 80%, 85%, 90%, 95%, 99% or 100% sequence identity to the amino acid sequence corresponding to amino acids 140-813 of SEQ ID NO: 1. As provided herein, where the protein "comprises" an amino acid sequence, further amino acids may be present in the protein. Where the protein "consists" of an amino acid sequence, no further amino acids are present. The term "antibacterial" as used herein defines that a compound (such as, e.g. a protein) is capable to destroy bacteria or inhibit the growth of bacteria. In other words, the antibacterial protein has activity against bacteria. In the present disclosure, the antibacterial protein has activity against *C*. *acnes.*

In some embodiments, an isolated antibacterial protein comprising an amino acid sequence of SEQ ID NO: 1 is provided. In some embodiments, the isolated antibacterial protein consists of an amino acid sequence of SEQ ID NO: 1. In some embodiments, an isolated antibacterial protein comprising an amino acid sequence having at least 80%, 85%, 90%, 95% or 99% sequence identity to the amino acid sequence of SEQ ID NO: 1 is provided. In some embodiments, the isolated antibacterial protein consists of an amino acid sequence having at least 80%, 85%, 90%, 95% or 99% sequence identity to the amino acid sequence of SEQ ID NO: 1. In yet further embodiments, an isolated antibacterial protein comprising an amino acid sequence having at least 80%, 85%, 90%, 95%, 99% or 100% sequence identity to the amino acid sequence corresponding to amino acids 140-813 of SEQ ID NO: 1 is provided.

The present invention disclosure further provides a composition for targeting *Cutibacterium acnes* comprising the antibacterial protein of the present invention. In some embodiments, the composition comprises said antibacterial protein at a concentration from about 100 ng/ml to about 1 mg/ml. For example, the antibacterial protein may be provided at a concentration of about 100 ng/ml, about 200 ng/ml, about 300 ng/ml, about 400 ng/ml, about 500 ng/ml, about 600 ng/ml, about 700 ng/ml, about 800 ng/ml, about 900 ng/ml, about 1 µg/ml, about 2 µg/ml, about 3 µg/ml, about 4 µg/ml, about 5 µg/ml, about 6 µg/ml, about 7 µg/ml, about 8 µg/ml, about 9 µg/ml, about 10 µg/ml, about 20 µg/ml, about 30 µg/ml, about 40 µg/ml, about 50 µg/ml, about 60 µg/ml, about 70 µg/ml, about 80 µg/ml, about 90 µg/ml, about 100 µg/ml, about 200 µg/ml, about 300 µg/ml, about 400 µg/ml, about 500 µg/ml, about 600 µg/ml, about 700 µg/ml, about 800 µg/ml, about 900 µg/ml, or about 1 mg/ml. Preferably, the antibacterial protein is provided at a concentration from about 100 ng/ml to about 100 µg/ml, more preferably at a concentration of about 100 µg/ml or of about 10 µg/ml. In some embodiments, the composition comprises the antibacterial protein at a concentration from 100 ng/ml to 1 mg/ml. In some embodiments, the composition comprises the antibacterial protein at a concentration from 100 ng/ml to 100 µg/ml, preferably at a concentration of 100 µg/ml. In other embodiments, the composition comprises the antibacterial protein at a concentration from 100 ng/ml to 10 µg/ml, preferably at a concentration of 10 µg/ml. The composition comprising the antibacterial protein of the present invention may be a liquid composition, such as a solution or a dispersion. The liquid composition may be an aqueous solution. The aqueous solution as said composition may contain a buffer. The buffer may be an inorganic or organic acid and/or salts thereof. The pH of the solution may generally be from 6.5 to 8.

The present inventions disclosure further provides a cosmetic, dermatological or pharmaceutical composition comprising the isolated antibacterial protein of the present invention or the composition the isolated antibacterial protein of the present invention. Such compositions are used to control or prevent the growth of *C*. *acnes.*

In some embodiments, the cosmetic, dermatological or pharmaceutical composition comprises said antibacterial protein at a concentration from about 100 ng/ml to about 1 mg/ml. For example, the antibacterial protein may be provided at a concentration of about 100 ng/ml, about 200 ng/ml, about 300 ng/ml, about 400 ng/ml, about 500 ng/ml, about 600 ng/ml, about 700 ng/ml, about 800 ng/ml, about 900 ng/ml, about 1 µg/ml, about 2 µg/ml, about 3 µg/ml, about 4 µg/ml, about 5 µg/ml, about 6 µg/ml, about 7 µg/ml, about 8 µg/ml, about 9 µg/ml, about 10 µg/ml, about 20 µg/ml, about 30 µg/ml, about 40 µg/ml, about 50 µg/ml, about 60 µg/ml, about 70 µg/ml, about 80 µg/ml, about 90 µg/ml, about 100 µg/ml, about 200 µg/ml, about 300 µg/ml, about 400 µg/ml, about 500 µg/ml, about 600 µg/ml, about 700 µg/ml, about 800 µg/ml, about 900 µg/ml, or about 1 mg/ml. Preferably, the antibacterial protein is provided at a concentration from about 100 ng/ml to about 100 µg/ml, more preferably at a concentration of about 100 µg/ml or of about 10 µg/ml. In some embodiments, the cosmetic, dermatological or pharmaceutical composition comprises the antibacterial protein at a concentration from 100 ng/ml to 1 mg/ml. In some embodiments, the composition comprises the antibacterial protein at a concentration from 100 ng/ml to 100 µg/ml, preferably at a concentration of 100 µg/ml. In other embodiments, the composition comprises the antibacterial protein at a concentration from 100 ng/ml to 10 µg/ml, preferably at a concentration of 10 µg/ml.

In some embodiments, said cosmetic, dermatological or pharmaceutical composition is in a form of a gel, a lotion or a cream. It is understood that a person skilled in the art knows the additives and other components that are needed in order to provide a composition in a form of a gel, a lotion or a cream. It is further provided, that said compositions, in addition to the presently disclosed antibacterial protein may comprise further active ingredients as needed, that are useful for the purpose of a specific composition.

In some embodiments, the pharmaceutical composition comprising the antibacterial protein and one or more pharmaceutically acceptable carrier is provided.

The cosmetic, dermatological or pharmaceutical compositions as provided herein can be used in a topical application. The term "topical application" is used to describe the administration of the compositions to an external surface of a patient or subject, e.g. on skin. The compositions may be applied several times during a time period and in amounts as is appropriate for the indication. The present disclosure yet further provides the antibacterial protein of the present invention or the composition comprising the antibacterial protein of the present invention for use in a method of treating or preventing infection with *Cutibacterium acnes.* That is, the antibacterial protein of the invention and the composition of the invention may be used in therapy. They may be used in the treatment or prevention of a bacterial infection, preferably of a bacterial infection by *C*. *acne.* Humans are preferred subjects for the therapy. In some embodiments, the antibacterial protein of the invention or the composition comprising the antibacterial protein of the invention for use in a method of treatment or prevention of acne vulgaris are provided. Preferably, humans are subjects that are to be treated.

Further, the present invention disclosure provides a method for inhibiting or preventing the growth of *Cutibacterium acnes* comprising the step of contacting the bacteria with the antibacterial protein of the invention or with the composition comprising the antibacterial protein of the invention. Further provided is a nucleic acid encoding the antibacterial protein of the invention. In some embodiments, a recombinant expression vector comprising said nucleic acid is provided.

In some embodiments, a host cell comprising the nucleic acid encoding the antibacterial protein of the invention or the recombinant expression vector comprising said nucleic acid is provided. In some embodiments, the host cell is a bacterial or eukaryotic cell. The eukaryotic cell may be a mammalian cell, a plant cell or a yeast cell. In some embodiments, a host cell is a plant cell. Further, a plant comprising the nucleic acid molecule encoding the antibacterial protein of the invention is provided. As the antibacterial protein of the invention may be produced by expression in plants or cells thereof, the composition may be a plant material or extract of the plant material, wherein the plant material may be a material from a plant having expressed the antibacterial protein, preferably *Nicotiana* or an edible plant.

A bacteriocin according to the invention may be produced by known methods of protein expression in a standard expression system. Depending on the expression system and host used, an appropriate codon optimization may be needed that is achieved using known methods and programs in the art.

### EXAMPLES

### Example 1

### Construction of expression vectors and antibacterial protein expression in plants.

By conducting the Genbank database research for Glycoside hydrolases coding domains in genomes of *C*. *acnes* or *C*. *acnes* infecting bacteriophages we have retrieved the sequence of a protein ALT33441. The sequence is coding for a protein of 813 a.a. (SEQ ID NO: 1) and contains GH25_bacA-like muramidase domain and PGRP-binding domain of peptidoglycan hydrolases (Fig. 1 and Fig. 2). We presumed this protein belonging to bacteriophage integrated into C. *acnes* genome, as in near proximity to it we have detected several other bacteriophage genes: P27 phage terminase small subunit protein, Phage primase, P4 family, phage antirepressor KIAC domain-containing protein.

Coding sequence of ALT33441 was optimized for expression in *Nicotiana benthamiana* by GeneArt software and synthetized by Thermo Fisher Scientific. The sequence was inserted as BsaI-BsaI fragment into the pICH29912 (assembled TMV-based MagnICON^{®} vector for cytosolic expression (Marillonnet et al., 2005)). The resulting plasmid was used to transform *Agrobacterium tumefaciens* GV3101.

*N. benthamiana* plants were grown in a growth chamber at 25°C with a 16 h light and 8 h dark photoperiod. Five- to six-week-old plants were used for infiltration with recombinant *A*. *tumefaciens.*

*A. tumefaciens* cultures were grown overnight at 30 °C in Lysogeny broth (LB) medium containing 50 mg/l rifampicin and 50 mg/l kanamycin. *Agrobacterium* overnight cultures were adjusted to an OD₆₀₀ of 1.5, sedimented by centrifugation at 3220 × *g* for 5 min and resuspended in tap water. Overnight cultures were diluted 1:1000 starting from OD595=1.0 in tap water and supplemented with 0.05 % Silwet L77 (Kurt Obermeier). *Agrobacterium* suspension was poured into a desiccator vessel, connected to a vacuum pump. The entire leaf system of a plant was then submerged into the suspension. Agroinfiltration was achieved by applying (till pressure of 200 mbar) and releasing vacuum through the pump. Plant leaves were harvested 5-6 days post agroinfiltration.

### Expression in N. benthamiana

Five days after plant infiltration with *Agrobacterium tumefaciens* strain carrying the pICH29912-ALT33441 expression vector, *N. benthamiana* leaves were used for extraction of total soluble protein by using two extraction buffers with different pH (pH 5 and pH 8). The extracts were analyzed by SDS-PAGE. Both extracts of infiltrated plants contained an additional band of approximately expected size (89 kDa), which is absent in wild-type non-infiltrated leaf extracts. The concentration of recombinant ALT33441 in extract was about 0.4 mg/ml (Fig. 3).

### Example 2

### Lytic activity detection in agar diffusion assays.

To prepare *C*. *acnes* lawns, bacterial strains were streaked from frozen stocks onto BHI agar and grown 4 days at 37 °C anaerobically. Liquid cultures were started by inoculating bacteria from fresh plates into 5 ml BHI and grown at 37 °C anaerobically for 3 days. Undiluted bacterial cultures were plated by using a sterile swab on fresh BHI plates. To prepare *S*. *epidermidis* lawns, bacterial cultures were grown in 5 ml of Tryptone soya yeast extract (TSYE) broth at 37 °C aerobically overnight. Undiluted bacterial culture was plated on TSYE agar by using a sterile swab. Crude protein extracts were prepared by using a buffer containing 50 mM sodium phosphate and 300 mM NaCl, pH 5.0. Images of plates were taken following two days anaerobic incubation at 37 °C. Next crude extracts were used for detection of lytic activity in agar diffusion assay. 8 C. *acnes* strains (ATCC-6921, ATCC-6922, ATCC-6923, ATCC-11827, ATCC-11828, ATCC-29399, ATCC-33179, ATCC-51277) and 3 *Staphylococcus epidermidis* strains (DSM1798, DSM20042, DSM20044) were used for preparing bacterial lawns. Sterile paper discs were placed on the surface of prepared plates and 20 µl of crude protein extract was applied. Plates were analyzed after three days anaerobic incubation at 37 °C. 7 out of 8 plates had large inhibition zones detected on the bacterial lawn where crude extract of ALT33441 was applied (Fig. 4A) demonstrating that ALT33441 has lytic activity against *C*. *acnes.* The sole *C*. *acnes* strain resistant to ALT33441, ATCC51277, was isolated not from human skin, but as an anaerobic culture contaminant and its involvement in skin colonization and acne vulgaris pathogenesis is questionable. None of three tested *S*. *epidermidis* strains was sensitive to ALT33441 inhibition zones (Fig 4B), demonstrating that the ALT33441 protein is inactive against this common bacterial colonizer of the skin. As a result, the inventors surprisingly found that ALT33441 is an active protein and that it is capable of lysing bacteria from the outside.

### Example 3

### Purification of antibacterial protein

A small portion of frozen leaf tissue was homogenized with chilled mortar and pestle in liquid nitrogen. Prepared powder was mixed with buffer (50 mM NaH₂PO₄/Na₂HPO₄, pH 7.0) at a ratio of 1 g of plant material to 5 ml of buffer. The crude extract solution was mixed for 15-20 min at +20-25 °C temperature. Cell debris was removed by centrifugation at 3220 g, at 20 °C for 20 min. Pellets were discarded and the supernatant was filtered by passing solution through membrane filter (pore size 0.45 µm). Ammonium sulphate was added up to 0.5 M and the pH of solution adjusted to 7. The formed precipitate removed by centrifugation at 3220 *g*, at 20 °C for 5 min. The supernatant was taken as total soluble protein and applied for purification in two steps.

At the first purification step the chromatography column was filled with Phenyl sepharose FF resin (GE Healthcare Life Sciences, Uppsala, Sweden) and pre-equilibrated with buffer (50 mM NaH₂PO₄/Na₂HPO₄, 0.5 M (NH₄)₂SO₄, pH 7.0). Protein solution was loaded to column and the Phenyl sepharose bound protein fraction was eluted by washing with elution buffer (50 mM NaH₂PO₄/Na₂HPO₄, 0.3 M (NH₄)₂SO₄, pH 7.0). The collected protein fraction replaced to the diafiltrating concentrator (30 kDa) and centrifuged at 3220 g until the volume of protein solution decreased 8-10 folds. The concentrate was diluted up to a primary volume with buffer containing 50 mM NaH₂PO₄/Na₂HPO₄ (pH 8.0). Procedure was repeated till conductivity decreased below 9 mS/cm and protein solution subjected to the final purification step using Q sepharose FF resin (GE Healthcare Life Sciences, Uppsala, Sweden). Chromatography media was pre-equilibrated with buffer (50 mM NaH₂PO₄/Na₂HPO₄, pH 8.0). Protein solution was loaded to column, washed with buffer (50 mM NaH₂PO₄/Na₂HPO₄, pH 8.0) and the target protein collected in flow through fraction (Fig 5). The purified protein was of about 95% homogeneity.

### Example 4

### MIC and MBC determination

Bacterial culture was grown in 5 ml Brain Heart Infusion (BHI) at 37 °C anaerobically. Bacterial culture was diluted 100 x: 5 µl of bacteria mixed with 495 µl BHI. Each well of 96-well plate contained 5 µl of 100 × diluted bacteria, 85 µl of BHI and 10 µl of protein. Purified ALT33441 protein (0.64 µg/µl) was diluted by combining 30 µl of purified protein with 30 µl BHI. Control samples contained BHI instead of protein. Control CFU/ml samples were prepared by mixing 5 µl of 100 × diluted bacterial culture with 95 µl BHI and further diluting up to 1000 times. 50 µl was plated on BHI and incubated anaerobically at 37 °C for three days. 96-well plate was incubated anaerobically at 37 °C for three days as well. 50 µl of undiluted or diluted sample was plated on BHI. Dilution was done by combining 50 µl of sample with 450 µl of BHI. Images of plates were taken following three days anaerobic incubation at 37 °C. Minimal inhibitory concentration (MIC) was determined by eye. Minimal bactericidal concentration (MBC) was determined by CFU/ml counting and represented protein concentration that was able to kill >99,9 % of initial bacterial inoculum.

### Results

Purified ALT33441 was used for determination of minimal inhibitory and minimal bactericidal concentration against a collection of *C*. *acnes* strains. It was observed that ALT33441 has extremely low MICs and MBCs against all tested sensitive strains. The determined MIC range was MIC 0.2-2 ng/ml and determined MBC range - 0.2-8 ng/ml. (Table 1). The resistant strain DSM16379 has been isolated not from the human skin, but as a contaminant of anaerobic culture and thus its ability to colonize skin or participate in the pathogenesis of acne is unknown.

**Table 1. Determined ALT33441 MIC and MBC against C. acnes strains.**

| *C*. *acnes* strain | MIC | MBC |
|---|---|---|
| | ng/ml | ng/ml |
| DSM16379 | no | no |
| DSM1897 | 2 | 4 |
| DSM30753 | 1 | 2 |
| DSM30738 | 2 | 4 |
| ATCC-6921 | 2 | 4 |
| ATCC-6922 | 2 | 8 |
| ATCC-6923 | 2 | 8 |
| ATCC-11827 | 1 | 2 |
| ATCC-11828 | 1 | 1 |
| ATCC12930 | 1 | 8 |
| ATCC-29399 | 1 | 4 |
| ATCC33179 | 2 | n.d. |

These surprisingly low concentrations demonstrating high antibacterial activity of ALT33441 are in sharp contrast with the data obtained with previously described *C*. *acnes* bacteriophage lytic proteins. To our knowledge, to the present date only two recombinantly expressed *C*. *acnes* bacteriophage lytic proteins have been described. There is some data showing some activity of these lysins against *C*. *acnes,* however, the described activity is week or moderate - the amounts of these lysins in microgram and even milligram order was needed to affect the growth of *C*. *acnes.* For example, purified recombinant CAP10-3 endolysin showed antimicrobial activity against *C*. *acnes* in the turbidity reduction test. Namely, the control group using the elution buffer reduced optical density at 600 nm of frozen C. *acnes* from 0.95 to 0.64, whereas 44, 87, 175 µg/mL of purified recombinant endolysin reduced it from 0.85 to 0.59, 0.92 to 0.45, and 0.94 to 0.35, respectively with a dose-dependent manner (Kim et al. 2023). In another study, endolysin PaAmi1 was evaluated for growth inhibition of *C*. *acnes* and 50 µg treatment has almost no effect, 1 mg of endolysin was necessary to achieve significant growth inhibition (Varotsou et al. 2023). No MIC or MBC data are available for CAP10-3 nor for PaAmi1 lysins.

Besides bacteriocins, different biologic substances have been recently evaluated for the capacity to inhibit or kill *C*. *acnes.* In recent study, a novel bacteriocin, HA2-5, from *Bacillus haynesii* HA2 was described, which effectively killed *P. acnes* through membrane disruption at a minimum inhibitory concentration (MIC) of 8 µg/mL (Peng et al .2023)

Prodigiosin, a red pigment produced by *Hahella chejuensis*, a marine-derived microorganism, has several biological functions, including antimicrobial activity and inflammatory relief. The MIC and MBC tests were performed to quantify the antibacterial activity of prodigiosin. All *Cutibacterium* strains had MIC of 25 µg/ml and slightly different MBCs (Kim et 1. 2023). Looking at the literature data outside of *Cutibacterium* lysins or bacteriocins, one of best performing bacteriophage lytic proteins, exebacase, has MICs against *S*. *aureus* starting from 0.25 µg/mL (Oh et al. 2021). In the light of all these results, ALT33441 MICs of 1-8 ng/ml range are exceptionally low.

### Example 5

### Human volunteer study

2 human volunteers with acne on the face or upper back participated in the study with the aim of skin bacteria isolation and testing of susceptibility to ALT33441 (qualitative test). The subject F22Y had mild acne on the face and severe acne on upper back and swabs for bacteria isolation were taken from the face and from the back. The subject M26Y had mild acne on the upper back and swab for bacteria isolation was taken from the back.

Bacterial cultures were grown in 5 ml BHI at 37 °C anaerobically for 3 days. Undiluted bacterial culture was plated by using a sterile swab on fresh BHI plates. Sterile paper discs were placed on the surface of prepared plates and 5 µl (5.5 µg) of purified ALT33441 was applied. 5 strains were subjected to DNA isolation and 16S rRNA coding gene sequencing.

### Results

In total 28 separate anaerobically grown colonies were isolated. 9 have been identified as facultative anaerobes, as they also grew in aerobic conditions. 11 M26Y strains were strict anaerobes and 10 between them were sensitive to ALT33441. Two sensitive and one insensitive strain were subjected to 16S rRNA gene sequencing. Both sensitive strains were identified as *C*. *acnes.* The insensitive strain was identified as *C*. *granulosum.* 8 F22Y strains were strict anaerobes and 5 of them were sensitive to ALT33441. Two sensitive and two insensitive strains were subjected to 16S rRNA gene sequencing. Both sensitive strains were confirmed to be *C*. *acnes.* Both insensitive anaerobe strains were identified as *S*. *saccharolyticus.*

**Table 2. Susceptibility of isolated skin bacteria to ALT33441.**

| | Anaerobic growth | Aerobic growth | Susceptible to ALT33441 | Sequencing |
|---|---|---|---|---|
| F22Y | | | | |
| 1 | + | + | No | n.t. |
| 2 | + | + | No | n.t. |
| 3 | + | + | No | n.t. |
| 4 | + | + | No | n.t. |
| 5 | + | | Yes | *C*. *acnes* |
| 6 | + | | Yes | *C*. *acnes* |
| 7 | + | | Yes | n.t. |
| 8 | + | | Yes | n.t. |
| 9 | + | + | No | n.t. |
| 10 | + | | No | *S*. *saccharolyticus* |
| 11 | weak | | No | n.t. |
| 12 | + | | No | *S*. *saccharolyticus* |
| 13 | + | + | No | n.t. |
| 14 | + | + | No | n.t. |
| 15 | + | + | No | n.t. |
| 16 | + | | Yes | n.t. |
| M26Y | | | | |
| 1 | + | + | No | n.t. |
| 2 | + | | Yes | *C*. *acnes* |
| 3 | + | | No | *C*. *granulosum* |
| 4 | + | | Yes | *C*. *acnes* |
| 5 | + | | Yes | n.t. |
| 6 | + | | Yes | n.t. |
| 7 | + | | Yes | n.t. |
| 8 | + | | Yes | n.t. |
| 9 | + | | Yes | n.t. |
| 10 | + | | Yes | n.t. |
| 11 | + | | Yes | n.t. |
| 12 | + | | Yes | n.t. |

| | | | | |
|---|---|---|---|---|
| *n.t. - not tested | | | | |

### Example 6

### Antibacterial activity of ALT33441 after storage at different temperatures

ALT33441 was purified as described (Example 4). Protein solution in 50 mM phosphate buffer pH 8.0 was adjusted to concentration of 1 mg/ml and aliquoted to 100 µl portions in microcentrifuge tubes. For stability tests, four series of samples were prepared: for storage at room temperature, for storage at 4 °C, at 37 °C and at 40 °C. Stability analysis at room temperature and at 4 °C was caried out for up to 13 months, and stability analysis at 37 °C and 40 °C up to 36 days. For ALT33441 antibacterial activity assay, *C*. *acnes* DSM1897 was inoculated from a frozen stock and grown on BHI agar at 37 °C for 3 days anaerobically. It was used to start a liquid bacterial culture in 5 ml BHI. Liquid culture was grown at 37 °C for 3 days anaerobically. Undiluted bacterial culture was spread on BHI agar by using a sterile cotton swab. Sterile paper discs were placed on top of spread bacteria and 5 µl of protein solution was applied. Serial two-fold dilutions were done by diluting the protein solution with 50 mM phosphate buffer pH 8.0. Images were taken following incubation at 37 °C for 3 days anaerobically.

### Results

The lowest concentration of freshly purified protein that results in detectable inhibition zone on DSM1897 lawn is 1.6 µg/ml. After incubation for 13 months at 4 °C or at room temperature, this concentration is four times higher and equal to 6.2 µg/ml (Fig. 6). Thus, after more than one year at room temperature, the protein remains highly active, although the antibacterial activity is slightly diminished.

Activity assays of ALT33441 stored at higher temperatures (37 °C and 40 °C) were carried out for a shorter span of time, 36 days. At the end of this period, ALT33441 stored at both temperatures retained high activity, lowest concentration resulting into detectable inhibition zone being 3.1 µl/ml and 6.2 µg/ml for 37 °C and 40 °C, respectively (Fig.6).

### References

Alexeyev OA, Marklund I, Shannon B, et al. Direct visualization of Propionibacterium acnes in prostate tissue by multicolor fluorescent in situ hybridization assay. Journal of Clinical Microbiology. 2007;45(11):3721-3728.

Brüggemann H, Food R. Bacteriophages infecting Propionibacterium acnes. Biomed Res Int. 2013;2013:705741. doi: 10.1155/2013/705741. Epub 2013 Apr 11. PMID: 23691509; PMCID: PMC3652107.

Dessinioti C, Katsambas A. Propionibacterium acnes and antimicrobial resistance in acne. Clin. Dermatol. 2017;35:163-167. doi: 10.1016/J.clindermatol.2016.10.008.

1Kim JI, Hasnain MA, Moon GS. Expression of a recombinant endolysin from bacteriophage CAP 10-3 with lytic activity against Cutibacterium acnes. Sci Rep. 2023 Sep 30;13(1):16430. doi: 10.1038/s41598-023-43559-z. PMID: 37777575; PMCID: PMC10542754.

2Kim HJ, Lee MS, Jeong SK, Lee SJ. Transcriptomic analysis of the antimicrobial activity of prodigiosin against Cutibacterium acnes. Sci Rep. 2023 Oct 13;13(1):17412. doi: 10.1038/s41598-023-44612-7. PMID: 37833344; PMCID: PMC10576067.

Kraft J, Freiman A. Management of acne. Can. Med. Assoc. J. 2011;183:E430-E435. doi: 10.1503/cmaj .090374

Lomholt HB, Kilian M. Population genetic analysis of Propionibacterium acnes identifies a subpopulation and epidemic clones associated with acne. PLoS ONE. 2010;5(8)e12277;

Oh JT, Ambler JE, Cassino C, Schuch R. Development of a Broth Microdilution Method for Exebacase Susceptibility Testing. Antimicrob Agents Chemother. 2021 Jun 17;65(7):e0258720. doi: 10.1128/AAC.02587-20. Epub 2021 Jun 17. Erratum in: Antimicrob Agents Chemother. 2021 Sep 17;65(10):e0165821. PMID: 33903102; PMCID: PMC8218677.

Peng Z, He M, Yang X, Zhang J. Discovery and Characterization of a Novel Bacteriocin HA2-5 that Strongly Inhibits Propionibacterium acnes. J Agric Food Chem. 2023 Aug 30;71(34):12741-12748. doi: 10.1021/acs.jafc.3c04617. Epub 2023 Aug 16. PMID: 37587448.

Varotsou C, Premetis GE, Labrou NE. Characterization and Engineering Studies of a New Endolysin from the Propionibacterium acnes Bacteriophage PAC1 for the Development of a Broad-Spectrum Artilysin with Altered Specificity. Int J Mol Sci. 2023 May 10;24(10):8523. doi: 10.3390/ijms24108523. PMID: 37239874; PMCID: PMC10218239.

## Claims

1. An isolated antibacterial protein comprising a polypeptide comprising or consisting of:
a) an amino acid sequence of SEQ ID NO: 1;
b) an amino acid sequence having at least 80%, 85%, 90%, 95% or 99% sequence identity to the amino acid sequence of SEQ ID NO: 1; or
c) an amino acid sequence having at least 80%, 85%, 90%, 95%, 99% or 100% sequence identity to the amino acid sequence corresponding to amino acids 140-813 of SEQ ID NO: 1.

2. A composition for targeting *Cutibacterium acnes* comprising the antibacterial protein according to claim 1.

3. The composition according to claim 2, wherein the antibacterial protein is provided at a concentration from about 100 ng/ml to about 1 mg/ml.

4. The composition according to claim 3, wherein the antibacterial protein is provided at a concentration from about 100 ng/ml to about 100 µg/ml, preferably at a concentration of about 100 µg/ml.

5. The composition according to claim 3, wherein the antibacterial protein is provided at a concentration from about 100 ng/ml to about 10 µg/ml, preferably at a concentration of about 10 µg/ml.

6. A cosmetic, dermatological or pharmaceutical composition comprising the isolated antibacterial protein according to claim 1 or the composition according to any one of claims 2 to 5.

7. The composition according to claim 6, wherein said composition is in a form of a gel, a lotion or a cream.

8. The pharmaceutical composition according to claim 6 or 7, wherein the pharmaceutical composition comprises one or more pharmaceutically acceptable carrier.

9. The antibacterial protein according to claim 1 or the composition according to any one of claims 2 to 8 for use in a method of treating or preventing infection with *Cutibacterium acnes.*

10. The antibacterial protein according to claim 1 or the composition according to any one of claims 2 to 8 for use in a method of treatment or prevention of acne vulgaris.

11. A method for inhibiting or preventing the growth of *Cutibacterium acnes* comprising the step of contacting the bacteria with the antibacterial protein according to claim 1 or with the composition according to any one of claims 2 to 8.

12. A nucleic acid encoding the protein according to claim 1.

13. A recombinant expression vector comprising the nucleic acid according to claim 12.

14. A host cell comprising the nucleic acid according to claim 12 or the recombinant expression vector of claim 13.
